# EUROPEAN PATENT APPLICATION

(11) **EP 4 023 161 A1**
(43) Date of publication of application: **06.07.2022**
(21) Application number: 20859326.9
(22) Date of filing: 10.02.2020
(51) Int. Cl.: A61B 10/00, A61N 5/06, A61B 5/00

(54) **TREATMENT SUPPORTING DEVICE AND IMAGE GENERATION METHOD**

(30) Priority: 27.08.2019 US 201962892058 P
(71) Applicant: Shimadzu Corporation, Nakagyo-ku Kyoto-shi Kyoto 604-8511 (JP)
(72) Inventor: ISHIKAWA, Akihiro, Kyoto-shi, Kyoto 604-8511 (JP)
(74) Representative: Müller-Boré & Partner Patentanwälte PartG mbB
(86) International application number: PCT/JP2020/005160
(87) International publication number: WO 2021/038913

(57) **Abstract**

A treatment support apparatus (100) includes an excitation light source (21) configured to irradiate a fluorescent substance (301) of a drug (300) administered into a cancer patient's (200) body with excitation light in a specific waveband having energy that excites the fluorescent substance (301) but does not kill a cancer cell (301) before or after treatment to kill the cancer cell (201) based on irradiating the drug (300) containing the fluorescent substance (301) with light in a specific waveband, a fluorescence detector (26) configured to detect fluorescence emitted by the fluorescent substance (301) of the drug (300) due to excitation by the excitation light, and an image generator (16) configured to generate a fluorescence distribution image (41), which is an image showing a distribution state of the fluorescence emitted by the fluorescent substance (301), based on the fluorescence from the fluorescent substance (301) detected by the fluorescence detector (26).

## Description

### Technical Field

The present invention relates to a treatment support apparatus and an image generation method, and more particularly, it relates to a treatment support apparatus and an image generation method in which a drug containing a fluorescent substance is irradiated with light in a specific waveband.

### Background Art

In recent years, photoimmunotherapy has been attracting attention as a new cancer treatment method. In photoimmunotherapy, a drug containing a fluorescent substance that causes a photochemical reaction and an antibody that selectively binds to cancer cells is first administered into the body of a cancer patient. The administered drug travels through the body of the cancer patient and selectively binds to an antigen of the cancer cells. Next, light in a specific waveband according to the fluorescent substance is radiated such that the fluorescent substance of the drug emits fluorescence and causes a photochemical reaction to change the chemical structure of the fluorescent substance. This change in the chemical structure of the fluorescent substance causes a change in the three-dimensional structure of the antibody. Then, the change in the three-dimensional structure of the antibody that has bound to the cancer cells damages the cell membranes of the cancer cells to which the antibody has bound to destroy (kill) the cancer cells.

Patent Document 1 discloses displaying an image showing the distribution state of fluorescence emitted by a fluorescent substance of a drug during treatment of photoimmunotherapy, i.e., when cancer cells are killed based on irradiating the drug that binds to the cancer cells with light in a specific waveband.

### Prior Art

### Patent Document

Patent Document 1: International Publication No. 2019/215905

### Summary of the Invention

### Problems to be Solved by the Invention

In photoimmunotherapy, in order to enhance the therapeutic effect, it is desirable to confirm the distribution state of the drug before the treatment and start the treatment in a state in which a sufficient amount of drug binds to the cancer cells in the body of a cancer patient. In addition, there is a desire to confirm the presence or absence of the drug that does not cause a photochemical reaction (does not act on the treatment) from the distribution state of the fluorescence emitted by the fluorescent substance of the drug in order to determine whether or not additional treatment is needed after the treatment. Therefore, it is desired to be able to confirm the distribution state of the drug that binds to the cancer cells even during non-treatment (before or after the treatment).

The present invention is intended to solve the above problems. The present invention aims to provide a treatment support apparatus and an image generation method, each of which enables the distribution state of a drug that binds to cancer cells to be confirmed before or after treatment to kill the cancer cells based on irradiating the drug that binds to the cancer cells with light in a specific waveband.

### Means for Solving the Problems

In order to attain the aforementioned object, a treatment support apparatus according to a first aspect of the present invention includes an excitation light source configured to irradiate a fluorescent substance of a drug administered into a body of a subject with excitation light in a specific waveband having energy that excites the fluorescent substance but does not kill a cancer cell before or after treatment to kill the cancer cell based on irradiating the drug containing the fluorescent substance with light in a specific waveband, a fluorescence detector configured to detect fluorescence emitted by the fluorescent substance of the drug due to excitation by the excitation light, and an image generator configured to generate a fluorescence distribution image, which is an image showing a distribution state of the fluorescence emitted by the fluorescent substance, based on the fluorescence from the fluorescent substance detected by the fluorescence detector. In the treatment to kill the cancer cell based on irradiating the drug containing the fluorescent substance with the light in the specific waveband, the drug is administered to the subject, and then the light in the specific waveband is continuously radiated such that energy to kill the cancer cell is given to the fluorescent substance of the drug. The fluorescence emitted by the fluorescent substance of the drug may include not only a visible light region but also an infrared light region.

In order to attain the aforementioned object, a treatment support apparatus according to a second aspect of the present invention includes an excitation light source configured to irradiate a fluorescent substance of a drug administered into a body of a subject with excitation light in a specific waveband having energy that excites the fluorescent substance but does not kill a cancer cell before or after treatment to kill the cancer cell based on irradiating the drug containing the fluorescent substance with light in a specific waveband, a fluorescence detector configured to detect fluorescence emitted by the fluorescent substance of the drug due to excitation by the excitation light, and a distribution information output configured to output information about a distribution state of the fluorescence emitted by the fluorescent substance based on the fluorescence from the fluorescent substance detected by the fluorescence detector.

In order to attain the aforementioned object, an image generation method according to a third aspect of the present invention includes irradiating a fluorescent substance of a drug administered into a body of a subject with excitation light in a specific waveband having energy that excites the fluorescent substance but does not kill a cancer cell before or after treatment to kill the cancer cell based on irradiating the drug containing the fluorescent substance with light in a specific waveband, detecting fluorescence emitted by the fluorescent substance of the drug due to excitation by the excitation light, and generating a fluorescence distribution image, which is an image showing a distribution state of the fluorescence emitted by the fluorescent substance, based on the detected fluorescence emitted by the fluorescent substance of the drug due to the excitation by the excitation light.

### Effect of the Invention

In the treatment support apparatus according to the first aspect of the present invention, as described above, the excitation light source is configured to radiate the excitation light in the specific waveband having energy that excites the fluorescent substance but does not kill the cancer cell before or after the treatment, and the image generator is configured to generate the fluorescence distribution image, which is an image showing the distribution state of the fluorescence emitted by the fluorescent substance. Accordingly, before or after the treatment, the fluorescence distribution image, which is an image showing the distribution state of the fluorescence emitted by the fluorescent substance, can be generated in a state in which the fluorescent substance is excited without killing the cancer cell (without proceeding with the treatment), and thus using the fluorescence distribution image generated by the image generator, the distribution state of the drug that binds to the cancer cell can be visually and easily confirmed. Consequently, it is possible to provide the treatment support apparatus that enables the distribution state of the drug that binds to the cancer cell to be confirmed before or after the treatment to kill the cancer cell based on irradiating the drug that binds to the cancer cell with the light in the specific waveband.

In the treatment support apparatus according to the second aspect of the present invention, as described above, the excitation light source is configured to radiate the excitation light in the specific waveband having energy that excites the fluorescent substance but does not kill the cancer cell before or after the treatment, and the distribution information output is configured to output the information about the distribution state of the fluorescence emitted by the fluorescent substance. Accordingly, before or after the treatment, the information about the distribution state of the fluorescence emitted by the fluorescent substance can be output in a state in which the fluorescent substance is excited without killing the cancer cell (without proceeding with the treatment), and thus using the information about the distribution state of the fluorescence output by the distribution information output, the distribution state of the drug that binds to the cancer cell can be confirmed. Consequently, it is possible to provide the treatment support apparatus that enables the distribution state of the drug that binds to the cancer cell to be confirmed before or after the treatment to kill the cancer cell based on irradiating the drug that binds to the cancer cell with the light in the specific waveband.

In the image generation method according to the third aspect of the present invention, as described above, before or after the treatment, the excitation light in the specific waveband having energy that excites the fluorescent substance but does not kill the cancer cell is radiated, and the fluorescence distribution image, which is an image showing the distribution state of the fluorescence emitted by the fluorescent substance, is generated. Accordingly, before or after the treatment, the fluorescence distribution image, which is an image showing the distribution state of the fluorescence emitted by the fluorescent substance, can be generated in a state in which the fluorescent substance is excited without killing the cancer cell (without proceeding with the treatment), and thus using the generated fluorescence distribution image, the distribution state of the drug that binds to the cancer cell can be visually and easily confirmed. Consequently, it is possible to provide the image generation method that enables the distribution state of the drug that binds to the cancer cell to be confirmed before or after the treatment to kill the cancer cell based on irradiating the drug that binds to the cancer cell with the light in the specific waveband.

### Brief Description of the Drawings

FIG. 1 is a perspective view showing a treatment support apparatus according to an embodiment of the present invention.
FIG. 2 is a block diagram showing the overall configuration of the treatment support apparatus according to the embodiment of the present invention.
FIG. 3 is a diagram showing an example of a fluorescence distribution image according to the embodiment of the present invention.
FIG. 4 is a diagram showing an example of a visible light image according to the embodiment of the present invention.
FIG. 5 is a diagram showing an example of a composite image according to the embodiment of the present invention.
FIG. 6 is a diagram showing an example of a composite image during treatment according to the embodiment of the present invention.
FIG. 7 is a first diagram for illustrating photoimmunotherapy.
FIG. 8 is a second diagram for illustrating photoimmunotherapy.
FIG. 9 is a diagram showing an example of the tendency of a decrease in fluorescence intensity with an increase in integrated energy amount.
FIG. 10 is a diagram showing an example of the irradiation intensity and irradiation time of excitation light radiated when the fluorescence distribution image is generated before or after the treatment by the treatment support apparatus according to the embodiment of the present invention.
FIG. 11 is a diagram showing an example of the irradiation intensity and irradiation time of excitation light radiated when a fluorescence distribution image is generated before or after treatment by a treatment support apparatus according to a first modified example of the embodiment of the present invention.
FIG. 12 is a block diagram showing the overall configuration of a treatment support apparatus according to a second modified example of the embodiment of the present invention.
FIG. 13 is a block diagram showing the overall configuration of a treatment support apparatus according to a third modified example of the embodiment of the present invention.

### Modes for Carrying Out the Invention

An embodiment embodying the present invention is hereinafter described on the basis of the drawings.

### (Configuration of Treatment Support Apparatus)

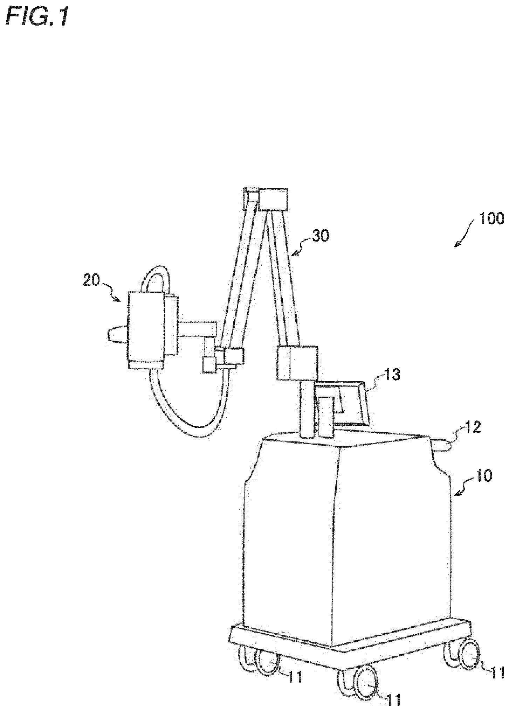

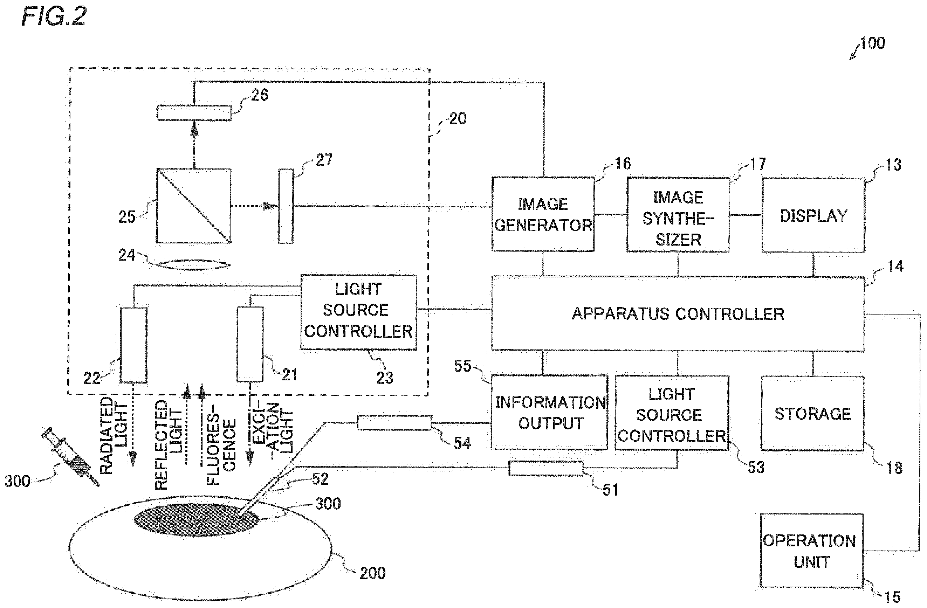

The configuration of a treatment support apparatus 100 according to the embodiment is now described with reference to FIGS. 1 and 2.

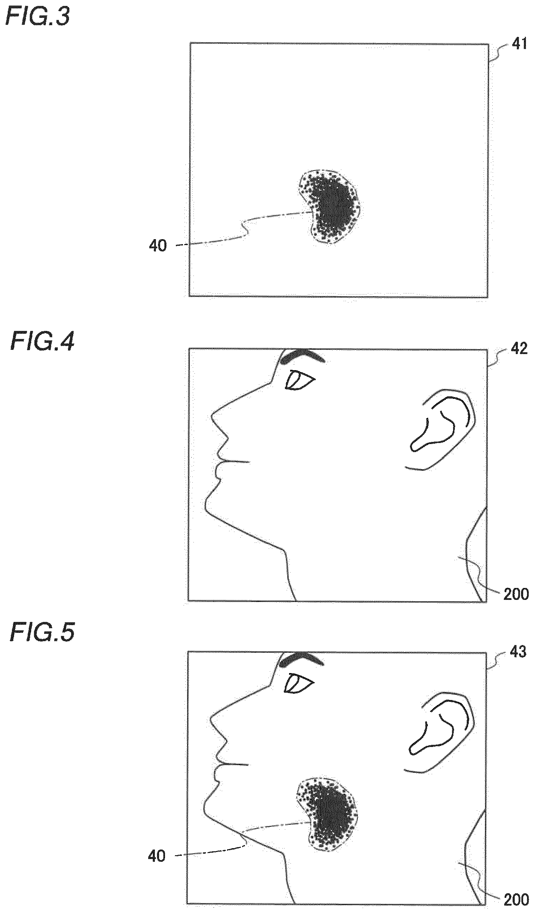

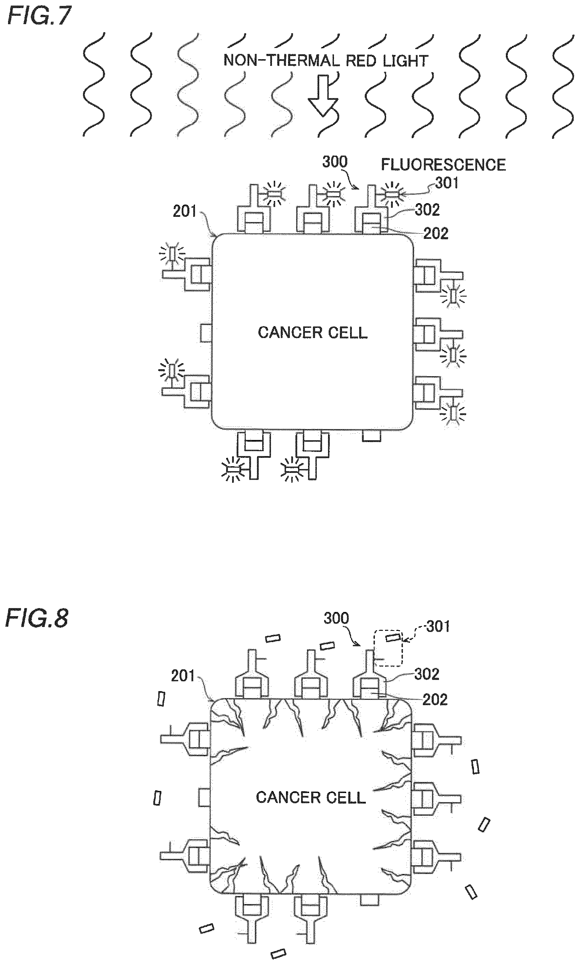

The treatment support apparatus 100 (see FIGS. 1 and 2) according to this embodiment is an apparatus that supports treatment of photoimmunotherapy. Specifically, the treatment support apparatus 100 is configured to generate a fluorescence distribution image 41 (see FIG. 3), which is an image showing a fluorescence distribution state, by irradiating a cancer patient 200 (see FIG. 2) with excitation light and detecting fluorescence (see FIG. 7) emitted by a fluorescent substance 301 (see FIGS. 7 and 8) of a drug 300 (see FIG. 2) administered into the body of the cancer patient 200. The cancer patient 200 is an example of a "subject" in the claims. The details of photoimmunotherapy are described below.

An operator such as a doctor can confirm, with the treatment support apparatus 100, how the drug 300 containing the fluorescent substance 301 is distributed in the body of the cancer patient 200 and how much the drug 300 is accumulated in the body of the cancer patient 200 before the treatment of photoimmunotherapy. In addition, the operator such as a doctor can grasp, with the treatment support apparatus 100, the effect of the treatment from the distribution and accumulation of the drug 300 containing the fluorescent substance 301 in the body of the cancer patient 200 after the treatment of the photoimmunotherapy.

The treatment support apparatus 100 is also configured to perform treatment to kill cancer cells 201 by photoimmunotherapy (treatment to kill the cancer cells 201 based on irradiating the drug 300 containing the fluorescent substance 301 with light in a specific waveband) by continuously radiating the light in the specific waveband according to the fluorescent substance 301, in addition to support for the treatment of photoimmunotherapy.

As shown in FIG. 1, the treatment support apparatus 100 includes an apparatus main body 10, an imager 20, and an arm unit 30. The apparatus main body 10 includes a plurality of wheels 11, a handle 12, and a display 13.

The imager 20 is connected to the apparatus main body 10 via the arm unit 30. The apparatus main body 10 is connected to one end of the arm unit 30, and the imager 20 is connected to the other end of the arm unit 30. The treatment support apparatus 100 is configured such that the excitation light irradiation direction (imaging direction) and the imaging position of the imager 20 can be adjusted by the arm unit 30.

Each of the plurality of wheels 11 is provided on a lower portion of the apparatus main body 10, and is configured to rotate.

The handle 12 is configured to be grippable, and is installed such that the operator such as a doctor can grip the handle 12 when moving the treatment support apparatus 100.

The display 13 is configured to display the fluorescence distribution image 41 (see FIG. 3), a visible light image 42 (see FIG. 4), and a composite image 43 (see FIG. 5), which are described below.

In this embodiment, the imager 20 of the treatment support apparatus 100 includes an excitation light source 21, a white light source 22, and a light source controller 23 (see FIG. 2). Furthermore, the imager 20 includes a lens 24, a prism 25, a fluorescence detector 26, and a visible light detector 27 (see FIG. 2).

The excitation light source 21 is configured to radiate excitation light in a specific waveband that excites the fluorescent substance 301 contained in the drug 300. The excitation light source 21 includes a semiconductor laser (LD: laser diode) or a light emitting diode (LED: light emitting diode), for example. The operator such as a doctor adjusts the excitation light irradiation direction of the imager 20 such that the excitation light of the excitation light source 21 is radiated from outside the body of the cancer patient 200 toward an affected area of the cancer patient 200. The specific waveband of the excitation light radiated when the fluorescent substance 301 contained in the drug 300 is excited may be the same as the specific waveband of the light (therapeutic light) radiated to the fluorescent substance 301 of the drug 300 during the treatment of photoimmunotherapy (the treatment to kill the cancer cells 201 based on irradiating the drug 300 containing the fluorescent substance 301 with the light in the specific waveband), or may be a waveband that does not partially overlap the specific waveband of the therapeutic light. Furthermore, the half-value width of the spectrum of the excitation light in the specific waveband radiated when the fluorescent substance 301 is excited may be different from the half-value width of the spectrum of the therapeutic light in the specific waveband.

The white light source 22 is a light source that emits visible light, and is configured to radiate white light. The white light source 22 includes a light emitting diode or a fluorescent lamp, for example. In order to detect visible light (reflected light) reflected from the cancer patient 200, the white light radiated from the white light source 22 is radiated from outside the body of the cancer patient 200 toward the affected area of the cancer patient 200.

The light source controller 23 is configured or programmed to control the excitation light source 21 to be turned on and off (to radiate the excitation light and stop radiating the excitation light). Furthermore, the light source controller 23 is configured or programmed to control the white light source 22 to be turned on and off (to radiation the excitation light and stop radiating the excitation light). The light source controller 23 is connected to an apparatus controller 14, and is configured or programmed to control irradiation with the excitation light by the excitation light source 21 and control irradiation with the white light by the white light source 22 based on an instruction from the apparatus controller 14. That is, the apparatus controller 14 controls irradiation with the excitation light by the excitation light source 21 and controls irradiation with the white light by the white light source 22 via the light source controller 23. The apparatus controller 14 is an example of a "controller" in the claims. The light source controller 23 and the apparatus controller 14 may be integrally configured.

The lens 24 is configured such that the fluorescence generated by the fluorescent substance 301 of the drug 300 and the visible light (reflected light) reflected by the cancer patient 200 are incident thereon. The fluorescence and the visible light incident on the lens 24 are converged by the lens 24, and are incident on the prism 25. The prism 25 is configured to separate the incident light, and the visible light and the fluorescence incident on the lens 24 are separated by the prism 25. The fluorescence separated by the prism 25 forms an image in the fluorescence detector 26. The visible light separated by the prism 25 forms an image in the visible light detector 27.

The fluorescence detector 26 is configured to detect fluorescence. The fluorescence detector 26 is an imaging device that detects the fluorescence emitted by the fluorescent substance 301 and separated by the prism 25. The fluorescence detector 26 images the fluorescence emitted by the fluorescent substance 301 by excitation with excitation light at the frame rate (30 frames/second (60 fields/second)) of National Television System Commission (NTSC) standards.

The visible light detector 27 is configured to detect visible light. The visible light detector 27 is an imaging device that detects the visible light (reflected light) reflected by the cancer patient 200 and separated by the prism 25. The visible light detector 27 images the visible light reflected from the cancer patient 200 at the frame rate (30 frames/second (60 fields/second)) of NTSC standards.

The apparatus controller 14 is electrically connected to the display 13, an operation unit 15, an image generator 16, an image synthesizer 17, a storage 18, the light source controller 23, a light source controller 53, and an information output 55 (see FIG. 2). The information output 55 is an example of a "distribution information output" in the claims.

The apparatus controller 14 includes a central processing unit (CPU), a read-only memory (ROM), a random access memory (RAM), etc., and is configured to control the entire treatment support apparatus 100.

The operation unit 15 is a user interface for operating the treatment support apparatus 100. For example, the operation unit 15 is configured to receive operations for controlling the treatment support apparatus 100 to turn on and off the excitation light source 21 (to radiate the excitation light and stop radiating the excitation light), to turn on and off the white light source 22 (to radiate the excitation light and stop radiating the excitation light), and to determine a display method for an image displayed on the display 13.

In this embodiment, the treatment support apparatus 100 includes the image generator 16 (see FIG. 2). A signal output by the fluorescence detector 26 based on the detected fluorescence and a signal output by the visible light detector 27 based on the detected visible light are input to the image generator 16. The image generator 16 is configured to generate an image based on the input signals. The image generator 16 is configured to generate the fluorescence distribution image 41 (see FIG. 3), which is an image showing the distribution state of the fluorescence emitted by the fluorescent substance 301, based on the fluorescence from the fluorescent substance 301 detected by the fluorescence detector 26. Furthermore, the image generator 16 is configured to generate the visible light image 42 (see FIG. 4) based on the visible light detected by the visible light detector 27.

The image synthesizer 17 is configured to generate the composite image 43 (see FIG. 5) in which the fluorescence distribution image 41 generated by the image generator 16 and the visible light image 42 generated by the image generator 16 are superimposed. That is, the image synthesizer 17 is configured to generate the composite image 43 in which a plurality of images generated by the image generator 16 are superimposed.

The apparatus controller 14 is configured or programmed to perform a control to display the composite image 43 on the display 13. The display 13 is configured to display the fluorescence distribution image 41 and the visible light image 42 in addition to the composite image 43 under the control of the apparatus controller 14. The display 13 may switchingly display any one of the fluorescence distribution image 41, the visible light image 42, and the composite image 43, or may display any two or all of the fluorescence distribution image 41, the visible light image 42, and the composite image 43 side by side at the same time.

The storage 18 is configured to store the fluorescence distribution image 41, the visible light image 42, the composite image 43, etc. In addition, the storage 18 is configured to store a program executed by the apparatus controller 14 to control excitation light irradiation and data required to control the excitation light irradiation when the fluorescence distribution image 41 is generated before or after the treatment.

The treatment support apparatus 100 includes an excitation light source 51, an optical fiber 52, the light source controller 53, a fluorescence detector 54, and the information output 55 (see FIG. 2).

The excitation light source 51 is configured to radiate excitation light in a specific waveband that excites the fluorescent substance 301 contained in the drug 300. The excitation light source 51 includes a semiconductor laser or a light emitting diode, for example.

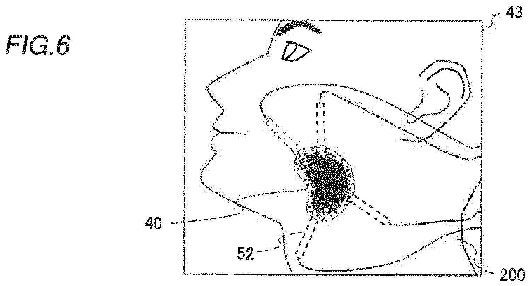

The optical fiber 52 is a composite-type (bidirectional) optical fiber 52 capable of guiding the light from the excitation light source 51 to the outside of the optical fiber 52 and guiding the light from the outside of the optical fiber 52 to the fluorescence detector 54. The optical fiber 52 is inserted into the body of the cancer patient 200 (see FIGS. 2 and 6). The optical fiber 52 is configured to guide and radiate the excitation light from the excitation light source 51 in the body of the cancer patient 200 to excite the fluorescent substance 301 of the drug 300 and guide the fluorescence generated from the fluorescent substance 301 to the fluorescence detector 54. The fluorescence generated by the fluorescent substance 301 excited by the excitation light radiated from the excitation light source 21 may be guided to the fluorescence detector 54 by the optical fiber 52. The treatment support apparatus 100 can also perform the treatment to kill the cancer cells 201 by continuously radiating the light in the specific waveband in the body of the cancer patient 200 (see FIGS. 2 and 6) using the optical fiber 52.

The light source controller 53 is configured or programmed to control the optical fiber 52 to be turned on and off (to radiate the excitation light and stop radiating the excitation light). Furthermore, the light source controller 53 is connected to the apparatus controller 14, and is configured or programmed to control irradiation with the excitation light in the body of the cancer patient 200 by the excitation light source 51 via the optical fiber 52 based on an instruction from the apparatus controller 14. The light source controller 53 and the apparatus controller 14 may be integrally configured, or the light source controller 53 and the light source controller 23 may be integrally configured.

The fluorescence detector 54 is configured to detect fluorescence. The fluorescence detector 54 is an imaging device that detects the fluorescence emitted by the fluorescent substance 301 and guided by the optical fiber 52. The fluorescence detector 54 images the fluorescence emitted by the fluorescent substance 301 at the frame rate (30 frames/second (60 fields/second)) of NTSC standards.

The information output 55 outputs information about the distribution state of the fluorescence emitted by the fluorescent substance 301 based on the fluorescence from the fluorescent substance 301 detected by the fluorescence detector 54.

The information output 55 outputs the information about the distribution state of the fluorescence emitted by the fluorescent substance 301 based on the fluorescence from the fluorescent substance 301 detected by the fluorescence detector 54. That is, the information output 55 is configured to output the information about the distribution state of the fluorescence based on information about the fluorescence guided by the optical fiber 52 and then detected by the fluorescence detector 54. Furthermore, the information output 55 may acquire positional information about the optical fiber 52 inserted into the body of the cancer patient 200 in addition to the information about the fluorescence detected by the fluorescence detector 54, and output the information about the distribution state of the fluorescence. The positional information about the optical fiber 52 may be acquired based on the visible light image 42. Alternatively, a sensor such as a motion sensor may be provided on the optical fiber 52, and the positional information about the optical fiber 52 may be acquired based on information such as acceleration detected by the sensor.

The information output 55 may be configured to output a detection of fluorescence intensity at or above a threshold as a sound, and notify the operator such as a doctor of the information about the distribution state of the fluorescence when the fluorescence intensity detected by the fluorescence detector 54 is equal to or more than a preset threshold. Furthermore, the information about the distribution state of the fluorescence output from the information output 55 may be displayed on the display 13 under the control of the apparatus controller 14.

### (Photoimmunotherapy)

Photoimmunotherapy in which the treatment support apparatus 100 supports the treatment is now described with reference to FIGS. 7 and 8.

In photoimmunotherapy, the drug 300 (see FIGS. 2, 7, and 8) is administered into the body of the cancer patient 200 (see FIGS. 2 and 4) before the treatment. The drug 300 contains the fluorescent substance 301 that emits fluorescence and an antibody 302 (see FIGS. 7 and 8).

The fluorescent substance 301 is a substance that is excited and emits fluorescence when irradiated with the light in the specific waveband. The fluorescent substance 301 is a chemical substance such as IRDye (registered trademark) 700DX, for example. IRDye 700DX is excited by light having a wavelength of 600 nm or more and 700 nm or less, and emits light having a wavelength of about 700 nm or 770 nm as fluorescence.

The fluorescent substance 301 is a substance that causes a photochemical reaction when continuously irradiated with the light in the specific waveband. During the treatment of photoimmunotherapy (the treatment that kills the cancer cells 201 based on irradiating the drug 300 containing the fluorescent substance 301 with the light in the specific waveband), the light in the specific waveband in which the fluorescent substance 301 causes a photochemical reaction according to the type of the fluorescent substance 301 of the drug 300 administered into the cancer patient 200 is radiated to the affected area of the cancer patient 200 (the fluorescent substance 301 of the drug 300 administered into the cancer patient 200). The light in the specific waveband radiated during the treatment is light in a waveband in which the fluorescent substance 301 of the drug 300 used for the treatment causes a photochemical reaction in a region from a portion of visible light to near-infrared light (600 nm or more and 2500 nm or less), and varies depending on the type of the fluorescent substance 301 of the drug 300 used for the treatment. When IRDye 700DX is used as the fluorescent substance 301, light having a wavelength of 600 nm or more and 700 nm or less, e.g., non-thermal red light having a wavelength of about 690 nm is radiated during the treatment of photoimmunotherapy (see FIG. 7).

The antibody 302 selectively binds to an antigen 202 of the cancer cells 201 (a specific protein expressed on the cancer cells 201). Therefore, the antibody 302 is selected according to the antigen 202 of the cancer cells 201 to be treated. The antibody 302 is an antibody such as cetuximab, for example.

The drug 300 administered into the cancer patient 200 enters the bloodstream, circulates in the body of the cancer patient 200, and selectively binds to the antigen 202 of the cancer cells 201 via the antibody 302. The fluorescent substance 301 of the drug 300 causes a photochemical reaction when continuously irradiated with the light in the specific waveband according to the fluorescent substance 301, and the chemical structure of the fluorescent substance 301 is changed. The change in the chemical structure of the fluorescent substance 301 causes a change in the three-dimensional structure of the antibody 302 (see FIG. 8). Then, the change in the three-dimensional structure of the antibody 302 that has bound to the cancer cells 201 damages the cell membranes of the cancer cells 201 (see FIG. 8) to which the antibody 302 has bound. Consequently, water invading from damaged portions of the cell membranes of the cancer cells 201 causes the cancer cells 201 to expand and rupture such that the cancer cells 201 are destroyed (killed). The fluorescent substance 301 does not emit fluorescence after the chemical structure thereof is changed by the photochemical reaction.

### (Observation of Fluorescence Distribution State)

Before the treatment, the operator such as a doctor can confirm how the drug 300 containing the fluorescent substance 301 is distributed in the body of the cancer patient 200 and how much the drug 300 is accumulated in the body of the cancer patient 200 by observing a fluorescence distribution 40 in the composite image 43 displayed on the display 13 or the fluorescence distribution 40 in the fluorescence distribution image 41.

The apparatus controller 14 is configured or programmed to perform a control to distinguishably display the visible light image 42 and the fluorescence distribution 40 which have been superimposed in the composite image 43 in order to allow the operator such as a doctor to easily confirm the fluorescence distribution 40 in the composite image 43. Specifically, the apparatus controller 14 performs a control to distinguishably display the visible light image 42 and the fluorescence distribution 40 by setting a display color of the fluorescence distribution 40 to a color that can be easily distinguished from the visible light image 42 (a fluorescent color such as green, purple, or blue, or a color different from the skin, tissue, and blood color of the cancer patient 200, for example) or blinking the fluorescence distribution 40, for example. The apparatus controller 14 may be configured or programmed to set a threshold for the detected fluorescence intensity or a total value of the detected intensity, for example, and control the display 13 to change a display method for the fluorescence distribution 40 or provide a display to notify that it has become equal to or more than the threshold when it has become equal to or more than the threshold.

The operator such as a doctor inserts the optical fiber 52 into the body of the cancer patient 200 for the treatment and starts the treatment to kill the cancer cells 201 based on radiating the light in the specific waveband. The fluorescent substance 301 of the drug 300 does not emit fluorescence after the chemical structure thereof is changed by the photochemical reaction, and thus the fluorescence distribution 40 changes as the treatment progresses. That is, the operator such as a doctor can grasp the progress of the treatment by the fluorescence distribution 40 in the composite image 43 (see FIG. 6) during the treatment or a change in the fluorescence distribution 40 in the fluorescence distribution image 41 during the treatment. After the treatment, the operator such as a doctor can grasp a distribution of the drug 300 containing fluorescent substance 301 in the body of cancer patient 200, and the therapeutic effect from accumulation by confirming the fluorescence distribution 40 in the composite image 43 displayed on the display 13, or the fluorescence distribution 40 in the fluorescence distribution image 41 as before the treatment.

The light (therapeutic light) in the specific waveband radiated for the treatment may be radiated from the excitation light source 21 while also serving as the excitation light, or may be radiated from an apparatus different from the treatment support apparatus 100.

In this embodiment, before or after the treatment to kill the cancer cells 201 based on irradiating the drug 300 containing the fluorescent substance 301 with the light in the specific waveband, the excitation light source 21 irradiates the fluorescent substance 301 of the drug 300 administered into the body of the cancer patient 200 with the excitation light in the specific waveband with energy that excites the fluorescent substance 301 but does not kill the cancer cells 201 (irradiates the fluorescent substance 301 with the excitation light in the specific waveband having energy that does not kill the cancer cells 201).

The energy that does not kill the cancer cells 201 refers to the integrated amount of energy obtained by integrating the irradiation intensity of the excitation light and the irradiation time of the excitation light. The energy that does not kill the cancer cells 201 varies depending on the site of the cancer and the drug 300 used for the treatment, for example, but can be confirmed in advance by a test or the like. For example, the states of the cancer cells 201 are observed with a microscope or the like while the cancer cells 201 to which the drug 300 has bound are irradiated with the light in the waveband in which the fluorescent substance 301 of the drug 300 causes a photochemical reaction such that the integrated amount of energy that does not kill the cancer cells 201 (does not cause the death of the cancer cells 201 to start) can be confirmed. As an example, in the case of head and neck cancer, when RM-1929 (ASP-1929) in which IRDye 700DX binds to cetuximab is used as the drug 300, the integrated amount of energy given during the treatment is 100 J/cm². On the other hand, the integrated amount of energy that does not kill the cancer cells 201 is about 0.5 J/cm², which is about 1/200 of the integrated amount of energy given during the treatment.

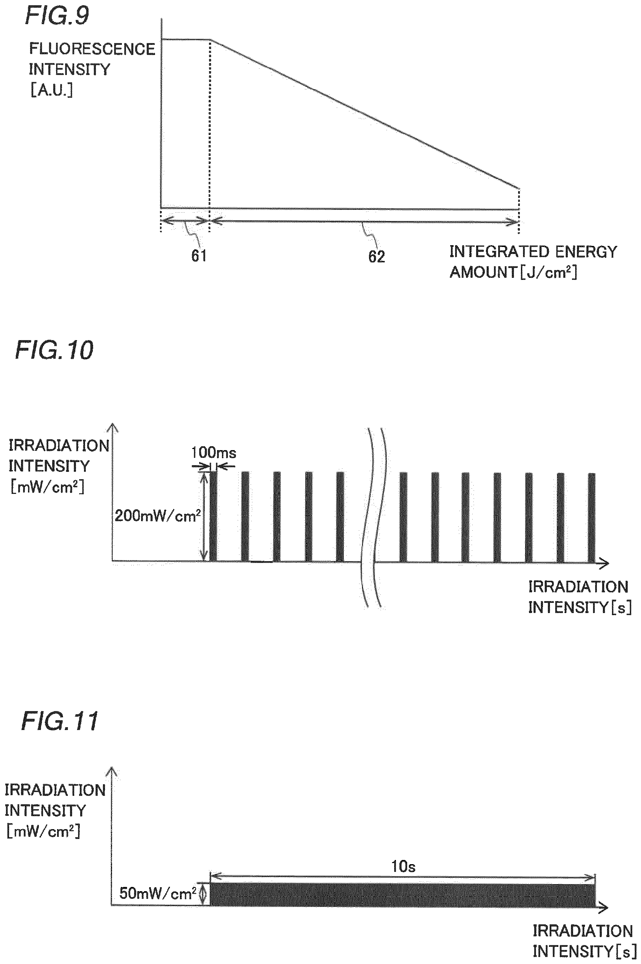

The apparatus controller 14 is configured or programmed to control irradiation with the excitation light by the excitation light source 21 such that the integrated amount of energy given to the fluorescent substance 301 by the excitation light is a first integrated energy amount 61 (see FIG. 9) when generating the fluorescence distribution image 41 before or after the treatment. The first integrated energy amount 61 is an integrated value of the irradiation intensity of the excitation light radiated to the fluorescent substance 301 when the fluorescence distribution image 41 is generated before or after the treatment and the irradiation time of the excitation light.

The excitation light source 21 is configured to radiate the excitation light such that the first integrated energy amount 61 (see FIG. 9), which is the integrated amount of energy given to the fluorescent substance 301 by the excitation light, is smaller than a second integrated energy amount 62 (see FIG. 9), which is the integrated amount of energy given to the fluorescent substance 301 by the light in the specific waveband during the treatment, when the fluorescence distribution image 41 is generated before or after the treatment. The second integrated energy amount 62 is an integrated value of the irradiation intensity of the light radiated to the fluorescent substance 301 during the treatment and the irradiation time of the radiated light.

As described above, the fluorescent substance 301 does not emit fluorescence after the chemical structure thereof is changed by the photochemical reaction. Therefore, as the treatment to kill the cancer cells 201 based on irradiating the drug 300 containing the fluorescent substance 301 with the light in the specific waveband progresses, the fluorescent substance 301 that emits fluorescence decreases. Furthermore, the first integrated energy amount 61 given to the fluorescent substance 301 by the excitation light radiated by the excitation light source 21 when the fluorescence distribution image 41 is generated is the integrated amount of energy in a range in which the rate of decrease in the fluorescence intensity (the vertical axis in FIG. 9) detected by the fluorescence detector 26 as the integrated amount of energy (the horizontal axis in FIG. 9) given to the fluorescent substance 301 increases is smaller than the rate of decrease in the fluorescence intensity during the treatment detected by the fluorescence detector 26 as the integrated amount of energy during the treatment given to the fluorescent substance 301 by the light in the specific waveband increases (see FIG. 9). Although the tendency of a decrease in the fluorescence intensity is shown linearly in FIG. 9, the decrease in the fluorescence intensity is not limited to a linear decrease, but the fluorescence intensity may decrease while repeatedly increasing and decreasing. Furthermore, the fluorescence intensity in the first integrated energy amount 61 is not limited to a constant value, but may increase or decrease.

The first integrated energy amount 61 is determined in a range equal to or less than the integrated energy amount obtained in advance, which does not kill the cancer cells 201 (does not cause the death of the cancer cells 201 to start). For example, when the integrated energy amount obtained in advance, which does not kill the cancer cells 201, is 0.5 J/cm², the first integrated energy amount 61 is determined in a range in which the integrated amount of energy is 0.5 J/cm² or less.

The magnitude of the first integrated energy amount 61 is equal to or more than magnitude that enables the fluorescent substance 301 to be excited, and is less than magnitude that causes the fluorescent substance 301 to change its chemical structure due to the photochemical reaction. That is, the magnitude of the first integrated energy amount 61 is magnitude at which the fluorescent substance 301 of the drug 300 emits fluorescence, and is magnitude at which the fluorescence emitted by the fluorescent substance 301 can be detected and the chemical structure of the fluorescent substance 301 is not changed. Therefore, the chemical structure of the fluorescent substance 301 is not changed, and thus the three-dimensional structure of the antibody 302 is not changed. In addition, the cancer cells 201 are not destroyed (killed) by the drug 300, and even if the cancer cells 201 are destroyed (killed), very few cancer cells 201 are destroyed (killed).

The irradiation intensity of the excitation light radiated when the fluorescence distribution image 41 is generated before or after the treatment is equal to or higher than the irradiation intensity of the light radiated during the treatment. For example, when the irradiation intensity of the light in the specific wavelength radiated during the treatment is 150 mW/cm², the irradiation intensity of the excitation light radiated when the fluorescence distribution image 41 is generated before or after the treatment is 150 mW/cm² or more. When the irradiation intensity of the light (therapeutic light) in the specific wavelength radiated during the treatment is 200 mW/cm², the irradiation intensity of the excitation light radiated when the fluorescence distribution image 41 is generated before or after the treatment is 200 mW/cm² or more.

The apparatus controller 14 is configured or programmed to control the excitation light source 21 to radiate excitation light with a predetermined pulse width based on the first integrated energy amount 61 when the fluorescence distribution image 41 is generated before or after the treatment. The apparatus controller 14 controls the excitation light source 21 to radiate the excitation light with the predetermined pulse width via the light source controller 23 each time the operator such as a doctor performs an operation. The fluorescence detector 26 is configured to detect fluorescence in synchronization with a predetermined pulse in which the excitation light is radiated. Therefore, the minimum value of the predetermined pulse width is changed based on a value of the speed of an imaging process in the fluorescence detector 26.

The apparatus controller 14 is configured or programmed to change settings of the irradiation time and the irradiation intensity of the excitation light source 21 such that the irradiation intensity of the excitation light radiated by the excitation light source 21 is maximized based on the number of times of imaging set (input) by the operator such as a doctor based on the first integrated energy amount 61. Specifically, when the number of times of imaging is 25 and the first integrated energy amount 61 is 0.5 J/cm² as described above, the irradiation time per irradiation is set to a minimum value, e.g., 100 ms, and the irradiation intensity of the excitation light radiated by the excitation light source 21 is set to a maximum value of 200 mW/cm² (see FIG. 10).

The storage 18 stores the first integrated energy amount 61 according to various treatment conditions such as the type of the excitation light source 21, the type of the drug 300, the type of the cancer, the site of the cancer, and information about the depth of the cancer, and the irradiation intensity that is optimum for generating the fluorescence distribution image 41 according to the treatment conditions.

The apparatus controller 14 may be configured or programmed to automatically set the irradiation intensity, the irradiation time, and the number of times of irradiation according to the treatment conditions based on the first integrated energy amount 61 and the optimum irradiation intensity according to the treatment conditions, for example, or may be configured or programmed such that the operator such as a doctor sets the irradiation intensity, the irradiation time, and the number of times of irradiation within a range that does not exceed the first integrated energy amount 61 according to the treatment conditions.

The apparatus controller 14 is configured or programmed to switch settings of the irradiation intensity, the irradiation time, and the number of times of irradiation of the excitation light by the method described above.

In this embodiment, the apparatus controller 14 is configured or programmed to perform a control to limit the irradiation intensity, the irradiation time, and the number of times of irradiation of the excitation light from the excitation light source 21 based on the first integrated energy amount 61. The apparatus controller 14 is configured or programmed to limit irradiation with the excitation light by the excitation light source 21 when the integrated amount of energy of the excitation light radiated during observation of the fluorescence distribution before or after the treatment is likely to exceed the integrated amount of energy determined based on the first integrated energy amount 61 in order to significantly reduce or prevent the progress of the treatment during the observation of the fluorescence distribution before or after the treatment (when the fluorescence distribution image 41 is generated before or after the treatment). The integrated amount of energy determined based on the first integrated energy amount 61 may be the same as the first integrated energy amount 61, and when it is equal to or more than the magnitude that enables the fluorescent substance 301 to be excited, it may be less than the first integrated energy amount 61.

For example, the apparatus controller 14 is configured or programmed to perform a control to limit irradiation with the excitation light such that the excitation light is not radiated even when the operator such as a doctor performs an operation when the number of times of irradiation that can be performed in the integrated amount of energy determined based on the first integrated energy amount 61 reaches an upper limit in the case in which the excitation light with the predetermined pulse width is radiated each time the operator such as a doctor performs an operation.

The apparatus controller 14 is configured or programmed to perform a control to limit irradiation with the excitation light by stopping irradiation with the excitation light (turning off the excitation light source 21) when the irradiation time of the excitation light is long and the integrated amount of energy given to the fluorescent substance 301 by the excitation light is likely to exceed the integrated amount of energy determined based on the first integrated energy amount 61 in a case in which the excitation light is continuously radiated.

The apparatus controller 14 is configured or programmed to perform a control to limit the irradiation intensity of the excitation light (reduce the irradiation intensity of the excitation light) when the irradiation intensity of the excitation light is high and the integrated amount of energy given to the fluorescent substance 301 by irradiation with the excitation light exceeds the integrated amount of energy determined based on the first integrated energy amount 61.

The limitation on the irradiation with the excitation light by the apparatus controller 14 may be released by the operation of the operator such as a doctor, if necessary. Furthermore, the control of the irradiation with the excitation light by the apparatus controller 14 as described above is performed not only on the excitation light source 21 but also on the excitation light source 51 via the light source controller 53, for example.

### Advantages of This Embodiment

In this embodiment, the following advantages are obtained.

In this embodiment, as described above, before or after the treatment, the excitation light source 21 irradiates the fluorescent substance 301 of the drug 300 with the excitation light in the specific waveband having energy that excites the fluorescent substance 301 but does not kill the cancer cells 201, and the image generator 16 generates the fluorescence distribution image 41, which is an image showing the distribution state of the fluorescence emitted by the fluorescent substance 301, based on the fluorescence from the fluorescent substance 301. Accordingly, before or after the treatment, the fluorescence distribution image 41, which is an image showing the distribution state of the fluorescence emitted by the fluorescent substance 301, can be generated in a state in which the fluorescent substance 301 is excited without killing the cancer cells 201 (without proceeding with the treatment), and thus using the fluorescence distribution image 41 generated by the image generator 16, the distribution state of the drug 300 that binds to the cancer cells 201 can be visually and easily confirmed. Consequently, it is possible to provide the treatment support apparatus 100 and an image generation method, each of which enables the distribution state of the drug 300 that binds to the cancer cells 201 to be confirmed before or after the treatment to kill the cancer cells 201 based on irradiating the drug 300 that binds to the cancer cells 201 with the light in the specific waveband.

In this embodiment, as described above, the excitation light source 21 radiates the excitation light such that the first integrated energy amount 61, which is the integrated amount of energy given to the fluorescent substance 301 by the excitation light, is smaller than the second integrated energy amount 62, which is the integrated amount of energy given to the fluorescent substance 301 by the light in the specific waveband during the treatment when the fluorescence distribution image 41 is generated before or after the treatment. Accordingly, when the fluorescence distribution image 41 is generated before or after the treatment, the integrated amount of energy given to the fluorescent substance 301 by the excitation light (first integrated energy amount 61) can be prevented from exceeding the integrated amount of energy given to the fluorescent substance 301 by the light in the specific waveband during the treatment (second integrated energy amount 62). Consequently, it is possible to prevent the treatment from being unintentionally started when the fluorescence distribution image 41 is generated before or after the treatment.

In this embodiment, as described above, the first integrated energy amount 61 is the integrated amount of energy in the range in which the rate of decrease in the fluorescence intensity detected by the fluorescence detector 26 as the integrated amount of energy given to the fluorescent substance 301 increases is smaller than the rate of decrease in the fluorescence intensity during the treatment detected by the fluorescence detector 26 as the integrated amount of energy during the treatment given to the fluorescent substance 301 by the light in the specific waveband increases. Accordingly, when the fluorescence distribution image 41 is generated, the rate of decrease in the fluorescence intensity detected by the fluorescence detector 26 as the integrated amount of energy increases is smaller than the rate of decrease in the fluorescence intensity during the treatment detected by the fluorescence detector 26 as the integrated amount of energy during the treatment given to the fluorescent substance 301 by the light in the specific waveband increases. Consequently, it is possible to confirm the distribution 40 of the fluorescence emitted by the fluorescent substance 301 of the drug 300 before the treatment progresses and the fluorescence intensity decreases.

In this embodiment, the magnitude of the first integrated energy amount 61 is equal to or more than the magnitude that enables the fluorescent substance 301 to be excited, and is less than the magnitude that causes the fluorescent substance 301 to change its chemical structure due to the photochemical reaction. Accordingly, the fluorescence detector 26 can detect the fluorescence emitted from the fluorescent substance 301 of the drug 300, and the death of the cancer cells 201 due to the photochemical reaction of the fluorescent substance 301 of the drug 300 caused by irradiation with the excitation light can be significantly reduced or prevented.

In this embodiment, as described above, the second integrated energy amount 62 is the integrated value of the irradiation intensity of the light radiated to the fluorescent substance 301 during the treatment and the irradiation time of the radiated light, and the first integrated energy amount 61 is the integrated value of the irradiation intensity of the excitation light radiated to the fluorescent substance 301 when the fluorescence distribution image 41 is generated before or after the treatment and the irradiation time of the excitation light. Furthermore, the apparatus controller 14 (controller) controls irradiation with the excitation light by the excitation light source 21 such that the integrated amount of energy given to the fluorescent substance 301 by the excitation light becomes the first integrated energy amount 61 when the fluorescence distribution image 41 is generated before or after the treatment. Accordingly, the first integrated energy amount 61 is based on the integrated value of the irradiation intensity of the excitation light radiated to the fluorescent substance 301 and the irradiation time of the excitation light, and thus when the fluorescence distribution image 41 is generated before or after the treatment, the irradiation intensity of the excitation light and the irradiation time of the excitation light are controlled such that the integrated amount of energy given to the fluorescent substance 301 by the excitation light can be easily controlled within the range of the first integrated energy amount 61. Consequently, the apparatus controller 14 can more easily control irradiation with the excitation light such that the first integrated energy amount 61 does not exceed the second integrated energy amount 62 when the fluorescence distribution image 41 is generated before or after the treatment.

In this embodiment, as described above, the apparatus controller 14 (controller) performs a control to limit the irradiation intensity, the irradiation time, and the number of times of irradiation of the excitation light from the excitation light source 21 based on the first integrated energy amount 61. Accordingly, it is possible to prevent the integrated amount of energy given to the fluorescent substance 301 by the excitation light from exceeding the first integrated energy amount 61 by increases in the irradiation intensity, the irradiation time, and the number of times of irradiation of the excitation light when the fluorescence distribution image 41 is generated before or after the treatment.

In this embodiment, the image generator 16 is configured to generate the visible light image 42 based on the visible light detected by the visible light detector 27, and the image synthesizer 17 is configured to generate the composite image 43 in which the fluorescence distribution image 41 and the visible light image 42 are superimposed. Furthermore, the apparatus controller 14 (controller) performs a control to display at least the composite image 43 on the display 13. Accordingly, the composite image 43 in which the fluorescence distribution image 41 before or after the treatment and the visible light image 42 are superimposed can be displayed on the display 13, and thus the fluorescence distribution image 41 and the visible light image 42 can be easily compared.

In this embodiment, as described above, the apparatus controller 14 (controller) controls the excitation light source 21 to radiate the excitation light with the predetermined pulse width based on the first integrated energy amount 61 when the fluorescence distribution image 41 is generated before or after the treatment. Accordingly, the excitation light with the pulse width based on the first integrated energy amount 61 is radiated, and thus the irradiation time of the excitation light radiated by the excitation light source 21 when the fluorescence distribution image 41 is generated before or after the treatment can be reduced. Consequently, the irradiation intensity of the excitation light can be increased within the range that does not exceed the first integrated energy amount 61.

In this embodiment, as described above, the irradiation intensity of the excitation light radiated when the fluorescence distribution image 41 is generated before or after the treatment is equal to or higher than the irradiation intensity of the light radiated during the treatment. Accordingly, when the fluorescence distribution image 41 is generated before or after the treatment, the excitation light with an irradiation intensity equal to the irradiation intensity of the light radiated during the treatment is radiated such that it is possible to confirm the distribution state of the drug 300 up to a depth position equivalent to a depth position reached by the light radiated during the treatment. When the fluorescence distribution image 41 is generated before or after the treatment, the excitation light with an irradiation intensity higher than the irradiation intensity of the light radiated during the treatment is radiated such that it is possible to confirm the distribution state of the drug 300 up to a depth position deeper than the depth position reached by the light radiated during the treatment.

In this embodiment, as described above, the excitation light source 51 irradiates the fluorescent substance 301 of the drug 300 with the excitation light in the specific waveband having energy that excites the fluorescent substance 301 but does not kill the cancer cells 201 before or after the treatment, and the information output 55 (distribution information output) outputs the information about the distribution state of the fluorescence emitted by the fluorescent substance 301 based on the fluorescence from the fluorescent substance 301. Accordingly, before or after the treatment, the information about the distribution state of the fluorescence emitted by the fluorescent substance 301 can be output in a state in which the fluorescent substance 301 is excited without killing the cancer cells 201 (without proceeding with the treatment), and thus using the information about the distribution state of the fluorescence output by the information output 55, the distribution state of the drug 300 that binds to the cancer cells 201 can be confirmed. Consequently, it is possible to provide the treatment support apparatus 100 that enables the distribution state of the drug 300 that binds to the cancer cells 201 to be confirmed before or after the treatment to kill the cancer cells 201 based on irradiating the drug 300 that binds to the cancer cells 201 with the light in the specific waveband.

### Modified Examples

The embodiment disclosed this time must be considered as illustrative in all points and not restrictive. The scope of the present invention is not shown by the above description of the embodiment but by the scope of claims for patent, and all modifications (modified examples) within the meaning and scope equivalent to the scope of claims for patent are further included.

For example, while the example in which the treatment support apparatus 100 includes the display 13 configured to display the composite image 43 has been shown in the aforementioned embodiment, the present invention is not limited to this. In the present invention, the treatment support apparatus may be configured to output a composite image or the like to a monitor or the like outside the apparatus, which displays the composite image or the like, without including the display.

While the example in which the irradiation intensity of the excitation light radiated when the fluorescence distribution image 41 is generated before or after the treatment is equal to or higher than the irradiation intensity of the light radiated during the treatment, and the excitation light source 21 is configured to irradiate the excitation light with the predetermined pulse width has been shown in the aforementioned embodiment, the present invention is not limited to this. In the present invention, as in a first modified example shown in FIG. 11, the irradiation intensity of the excitation light radiated when the fluorescence distribution image is generated before or after the treatment may be set to be lower than the irradiation intensity of the light radiated during the treatment (the irradiation intensity of the excitation light radiated when the fluorescence distribution image is generated before or after the treatment may be set to 50 mW/cm² when the irradiation intensity of the light in the specific wavelength radiated during the treatment is 200 mW/cm², for example), and the excitation light may be continuously radiated.

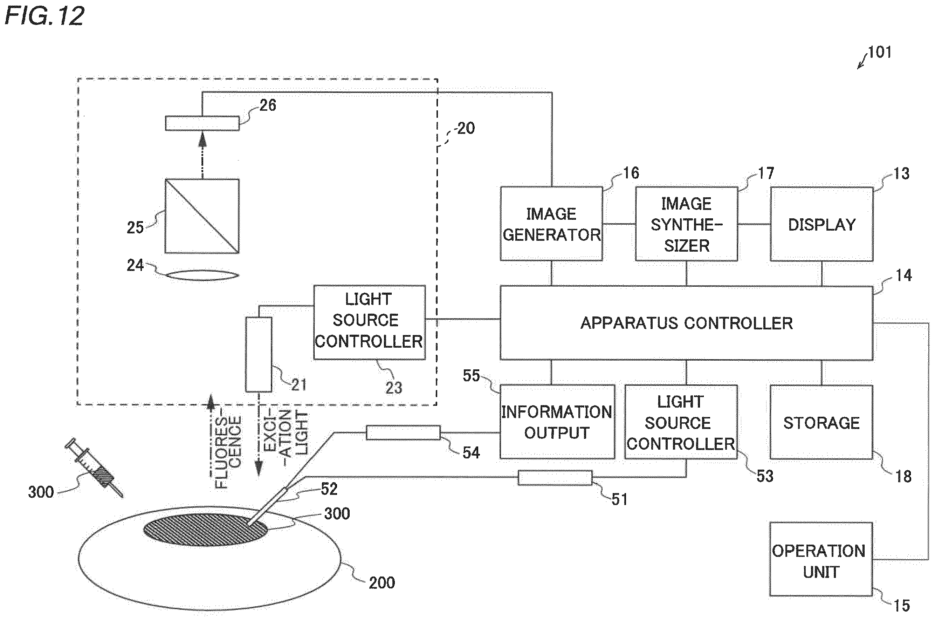

While the example in which the image generator 16 generates the visible light image 42 has been shown in the aforementioned embodiment, the present invention is not limited to this. In the present invention, as in a treatment support apparatus 101 according to a second modified example shown in FIG. 12, a white light source and a visible light detector may not be provided, and only a fluorescence distribution image may be generated by an image generator based on fluorescence from a fluorescent substance detected by a fluorescence detector.

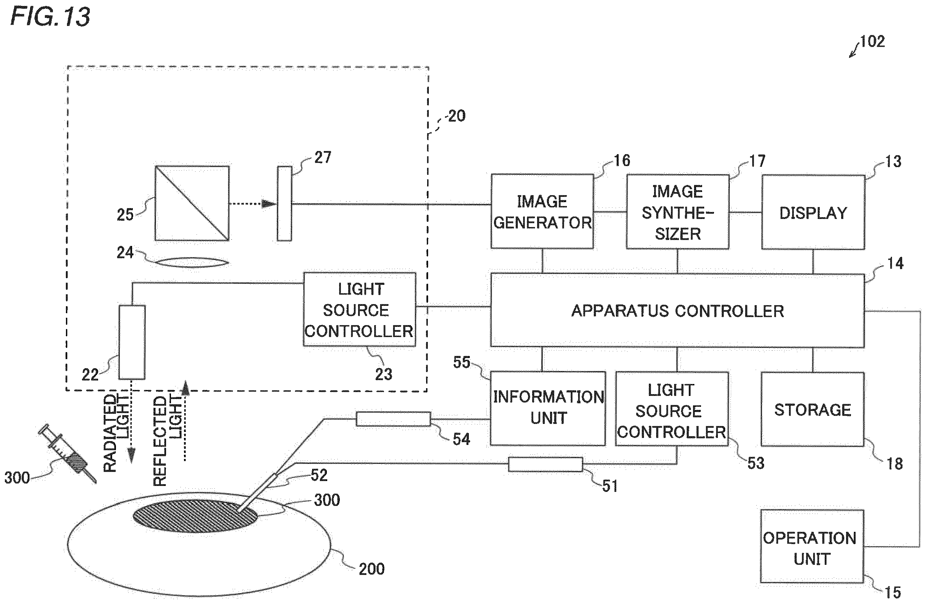

While the example in which the excitation light is radiated from outside the body of the cancer patient 200 by the excitation light source 21, and the fluorescence emitted by the fluorescent substance 301 is detected by the image generator 16 outside the body of the cancer patient 200 has been shown in the aforementioned embodiment, the present invention is not limited to this. In the present invention, as in a treatment support apparatus 102 according to a third modified example shown in FIG. 13, irradiation with excitation light and detection of fluorescence emitted by a fluorescent substance of a drug may be performed in the body of a cancer patient 200 via an optical fiber 52.

While the example in which the treatment support apparatus 100 is configured to perform treatment in addition to support for the treatment has been shown in the aforementioned embodiment, the present invention is not limited to this. In the present invention, the treatment support apparatus may be configured to only support the treatment of photoimmunotherapy (only generate the fluorescence distribution image or only output the information about the fluorescence distribution state).

### Aspects

It will be appreciated by those skilled in the art that the exemplary embodiments described above are specific examples of the following aspects.

### (Item 1)

A treatment support apparatus comprising:
an excitation light source configured to irradiate a fluorescent substance of a drug administered into a body of a subject with excitation light in a specific waveband with energy that excites the fluorescent substance but does not kill a cancer cell before or after treatment to kill the cancer cell based on irradiating the drug containing the fluorescent substance with light in a specific waveband;
a fluorescence detector configured to detect fluorescence emitted by the fluorescent substance of the drug due to excitation by the excitation light; and
an image generator configured to generate a fluorescence distribution image, which is an image showing a distribution state of the fluorescence emitted by the fluorescent substance, based on the fluorescence from the fluorescent substance detected by the fluorescence detector.

### (Item 2)

The treatment support apparatus according to item 1, wherein the excitation light source is configured to radiate the excitation light such that a first integrated energy amount, which is an integrated amount of energy given to the fluorescent substance by the excitation light, is smaller than a second integrated energy amount, which is an integrated amount of energy given to the fluorescent substance by the light in the specific waveband during the treatment when the fluorescence distribution image is generated before or after the treatment.

### (Item 3)

The treatment support apparatus according to item 2, wherein when the fluorescence distribution image is generated, the first integrated energy amount given to the fluorescent substance by the excitation light emitted by the excitation light source is an integrated amount of energy in a range in which a rate of decrease in a fluorescence intensity detected by the fluorescence detector as the integrated amount of the energy given to the fluorescent substance increases is smaller than a rate of decrease in a fluorescence intensity during the treatment detected by the fluorescence detector as the integrated amount of the energy during the treatment given to the fluorescent substance by the light in the specific waveband increases.

### (Item 4)

The treatment support apparatus according to item 2 or 3, wherein a magnitude of the first integrated energy amount is equal to or more than a magnitude that enables the fluorescent substance to be excited, and is less than a magnitude that causes the fluorescent substance to change a chemical structure thereof due to a photochemical reaction.

### (Item 5)

The treatment support apparatus according to any one of items 2 to 4, further comprising:
a controller configured or programmed to control irradiation with the excitation light by the excitation light source; wherein
the second integrated energy amount is an integrated value of an irradiation intensity of light radiated to the fluorescent substance during the treatment and an irradiation time of the radiated light;
the first integrated energy amount is an integrated value of an irradiation intensity of the excitation light radiated to the fluorescent substance when the fluorescence distribution image is generated before or after the treatment and an irradiation time of the excitation light; and
the controller is configured or programmed to control the irradiation with the excitation light by the excitation light source such that the integrated amount of the energy given to the fluorescent substance by the excitation light becomes the first integrated energy amount when the fluorescence distribution image is generated before or after the treatment.

### (Item 6)

The treatment support apparatus according to item 5, wherein the controller is configured or programmed to perform a control to limit an irradiation intensity, an irradiation time, and a number of times of irradiation of the excitation light from the excitation light source based on the first integrated energy amount.

### (Item 7)

The treatment support apparatus according to item 5 or 6, further comprising:
a visible light detector configured to detect visible light;
an image synthesizer configured to generate a composite image in which a plurality of images generated by the image generator are superimposed; and
a display configured to display the composite image; wherein
the image generator is configured to generate a visible light image based on the visible light detected by the visible light detector;
the image synthesizer is configured to generate the composite image in which the fluorescence distribution image and the visible light image are superimposed; and
the controller is configured or programmed to perform a control to display at least the composite image on the display.

### (Item 8)

The treatment support apparatus according to item 6 or 7, wherein the controller is configured or programmed to control the excitation light source to radiate the excitation light with a predetermined pulse width based on the first integrated energy amount when the fluorescence distribution image is generated before or after the treatment.

### (Item 9)

The treatment support apparatus according to item 8, wherein the irradiation intensity of the excitation light radiated when the fluorescence distribution image is generated before or after the treatment is equal to or higher than the irradiation intensity of the light radiated during the treatment.

### (Item 10)

A treatment support apparatus comprising:
an excitation light source configured to irradiate a fluorescent substance of a drug administered into a body of a subject with excitation light in a specific waveband having energy that excites the fluorescent substance but does not kill a cancer cell before or after treatment to kill the cancer cell based on irradiating the drug containing the fluorescent substance with light in a specific waveband;
a fluorescence detector configured to detect fluorescence emitted by the fluorescent substance of the drug due to excitation by the excitation light; and
a distribution information output configured to output information about a distribution state of the fluorescence emitted by the fluorescent substance based on the fluorescence from the fluorescent substance detected by the fluorescence detector.

### (Item 11)

An image generation method comprising:
irradiating a fluorescent substance of a drug administered into a body of a subject with excitation light in a specific waveband having energy that excites the fluorescent substance but does not kill a cancer cell before or after treatment to kill the cancer cell based on irradiating the drug containing the fluorescent substance with light in a specific waveband;
detecting fluorescence emitted by the fluorescent substance of the drug due to excitation by the excitation light; and
generating a fluorescence distribution image, which is an image showing a distribution state of the fluorescence emitted by the fluorescent substance, based on the detected fluorescence emitted by the fluorescent substance of the drug due to the excitation by the excitation light.

### Description of Reference Numerals

13: display
14: apparatus controller (controller)
16: image generator
17: image synthesizer
21: excitation light source
26: fluorescence detector
27: visible light detector
41: fluorescence distribution image
42: visible light image
43: composite image
51: excitation light source
54: fluorescence detector
55: information output (distribution information output)
61: first integrated energy amount
62: second integrated energy amount
100: treatment support apparatus
200: cancer patient (subject)
201: cancer cell
300: drug
301: fluorescent substance

## Claims

1. A treatment support apparatus comprising:
an excitation light source configured to irradiate a fluorescent substance of a drug administered into a body of a subject with excitation light in a specific waveband having energy that excites the fluorescent substance but does not kill a cancer cell before or after treatment to kill the cancer cell based on irradiating the drug containing the fluorescent substance with light in a specific waveband;
a fluorescence detector configured to detect fluorescence emitted by the fluorescent substance of the drug due to excitation by the excitation light; and
an image generator configured to generate a fluorescence distribution image, which is an image showing a distribution state of the fluorescence emitted by the fluorescent substance, based on the fluorescence from the fluorescent substance detected by the fluorescence detector.

2. The treatment support apparatus according to claim 1, wherein the excitation light source is configured to radiate the excitation light such that a first integrated energy amount, which is an integrated amount of energy given to the fluorescent substance by the excitation light, is smaller than a second integrated energy amount, which is an integrated amount of energy given to the fluorescent substance by the light in the specific waveband during the treatment when the fluorescence distribution image is generated before or after the treatment.

3. The treatment support apparatus according to claim 2, wherein when the fluorescence distribution image is generated, the first integrated energy amount given to the fluorescent substance by the excitation light emitted by the excitation light source is an integrated amount of energy in a range in which a rate of decrease in a fluorescence intensity detected by the fluorescence detector as the integrated amount of the energy given to the fluorescent substance increases is smaller than a rate of decrease in a fluorescence intensity during the treatment detected by the fluorescence detector as the integrated amount of the energy during the treatment given to the fluorescent substance by the light in the specific waveband increases.

4. The treatment support apparatus according to claim 2 or 3, wherein a magnitude of the first integrated energy amount is equal to or more than a magnitude that enables the fluorescent substance to be excited, and is less than a magnitude that causes the fluorescent substance to change a chemical structure thereof due to a photochemical reaction.

5. The treatment support apparatus according to any one of claims 2 to 4, further comprising:
a controller configured or programmed to control irradiation with the excitation light by the excitation light source; wherein
the second integrated energy amount is an integrated value of an irradiation intensity of light radiated to the fluorescent substance during the treatment and an irradiation time of the radiated light;
the first integrated energy amount is an integrated value of an irradiation intensity of the excitation light radiated to the fluorescent substance when the fluorescence distribution image is generated before or after the treatment and an irradiation time of the excitation light; and
the controller is configured or programmed to control the irradiation with the excitation light by the excitation light source such that the integrated amount of the energy given to the fluorescent substance by the excitation light becomes the first integrated energy amount when the fluorescence distribution image is generated before or after the treatment.

6. The treatment support apparatus according to claim 5, wherein the controller is configured or programmed to perform a control to limit an irradiation intensity, an irradiation time, and a number of times of irradiation of the excitation light from the excitation light source based on the first integrated energy amount.

7. The treatment support apparatus according to claim 5 or 6, further comprising:
a visible light detector configured to detect visible light;
an image synthesizer configured to generate a composite image in which a plurality of images generated by the image generator are superimposed; and
a display configured to display the composite image; wherein
the image generator is configured to generate a visible light image based on the visible light detected by the visible light detector;
the image synthesizer is configured to generate the composite image in which the fluorescence distribution image and the visible light image are superimposed; and
the controller is configured or programmed to perform a control to display at least the composite image on the display.

8. The treatment support apparatus according to claim 6 or 7, wherein the controller is configured or programmed to control the excitation light source to radiate the excitation light with a predetermined pulse width based on the first integrated energy amount when the fluorescence distribution image is generated before or after the treatment.

9. The treatment support apparatus according to claim 8, wherein the irradiation intensity of the excitation light radiated when the fluorescence distribution image is generated before or after the treatment is equal to or higher than the irradiation intensity of the light radiated during the treatment.

10. A treatment support apparatus comprising: an excitation light source configured to irradiate a fluorescent substance of a drug administered into a body of a subject with excitation light in a specific waveband having energy that excites the fluorescent substance but does not kill a cancer cell before or after treatment to kill the cancer cell based on irradiating the drug containing the fluorescent substance with light in a specific waveband;
a fluorescence detector configured to detect fluorescence emitted by the fluorescent substance of the drug due to excitation by the excitation light; and
a distribution information output configured to output information about a distribution state of the fluorescence emitted by the fluorescent substance based on the fluorescence from the fluorescent substance detected by the fluorescence detector.

11. An image generation method comprising:
irradiating a fluorescent substance of a drug administered into a body of a subject with excitation light in a specific waveband having energy that excites the fluorescent substance but does not kill a cancer cell before or after treatment to kill the cancer cell based on irradiating the drug containing the fluorescent substance with light in a specific waveband;
detecting fluorescence emitted by the fluorescent substance of the drug due to excitation by the excitation light; and
generating a fluorescence distribution image, which is an image showing a distribution state of the fluorescence emitted by the fluorescent substance, based on the detected fluorescence emitted by the fluorescent substance of the drug due to the excitation by the excitation light.
